# EUROPÄISCHE PATENTSCHRIFT

(11) **EP 0 642 303 B1**
(45) Veröffentlichungstag und Bekanntmachung des Hinweises auf die Patenterteilung: **02.08.2000**
(21) Anmeldenummer: 93910010.3
(22) Anmeldetag: 17.05.1993
(51) Int. Cl.: A01N 43/36, C07D 207/34

(54) **SUBSTITUIERTE 2-ARYLPYRROLE**
SUBSTITUTED 2-ARYLPYRROLES
2-ARYLPYRROLES SUBSTITUES

(30) Priorität: 29.05.1992 DE 4217722
(43) Veröffentlichungstag der Anmeldung: 15.03.1995
(73) Patentinhaber: BAYER AG, 51368 Leverkusen (DE)
(72) Erfinder: Uhr, Hermann, D-5090 Leverkusen 3 (DE); ERDELEN, Christoph, D-5653 Leichlingen 1 (DE); WACHENDORFF-NEUMANN, Ulrike, D-4019 Monheim (DE); STENDEL, Wilhelm, D-5600 Wuppertal 1 (DE)
(86) Internationale Anmeldenummer: EP9301231
(87) Internationale Veröffentlichungsnummer: WO9324001

(56) Entgegenhaltungen:
- EP-A- 0 312 723
- EP-A- 0 347 488
- EP-A- 0 358 047
- EP-A- 0 492 171
- EP-A- 0 515 941
- EP-A- 0 530 147
- EP-A- 0 530 515

## Beschreibung

Die vorliegende Erfindung betrifft neue substituierte 2-Arylpyrrole, Zwischenprodukte zu ihrer Herstellung und ihre Verwendung zur Bekämpfung von tierischen Schädlingen, insbesondere von Insekten, Spinnentieren und Nematoden, die in der Landwirtschaft, in den Formten, im Vorrats- und Materialschutz sowie auf dem Hygienesektor vorkommen.

Es ist bereits bekannt geworden, daß strukturell ähnliche Cyanopyrrole als Molluskizide, Fungizide und Insektizide wirksam sind (siehe dazu z.B. EP-A 0 347 488, EP-A 0 358 047, EP-A 0 312 723, DE-A 4 117 752). Die Wirksamkeit und Wirkungsbreite dieser Verbindungen ist jedoch insbesondere bei niedrigen Aufwandmengen und Konzentrationen nicht immer voll zufriedenstellend.

Es wurden nun neue substituierte 2-Arylpyrrole der allgemeinen Formel (I) 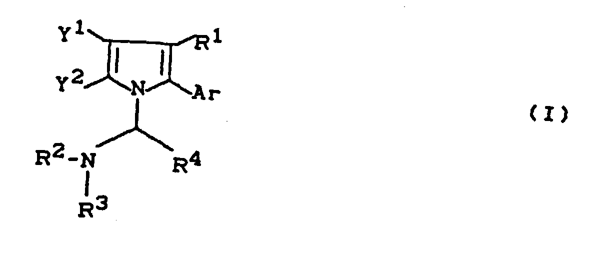 in welcher
- R¹: für Cyano oder Nitro steht,
- R² und R³: unabhängig voneinander entweder für Wasserstoff, C₁-C₈-Alkyl, C₃-C₈-Alkenyl oder C₃-C₈-Alkinyl stehen, wobei die Alkyl-, Alkenyl- oder Alkinylreste gegebenenfalls durch 1 bis 6 gleiche oder verschiedene Halogenatome, C₁-C₆-Alkoxy, C₁-C₆-Alkylthio, (C₁-C₆-Alkyl)carbonyloxy), (C₁-C₆-Alkoxy)-carbonyl, Phenyl, Cyano oder Nitro substituiert sind, oder für (C₁-C₈-Alkoxy)-carbonyl, (C₃-C₈-Alkenoxy)-carbonyl oder (C₃-C₈-Alkinoxy)-carbonyl stehen, wobei der Alkoxy-, Alkenoxy- oder Alkinoxyteil gegebenenfalls durch 1 bis 6 gleiche oder verschiedene Reste der Reihe Halogen, C₁-C₆-Alkoxy, C₁-C₆-Alkylthio, (C₁-C₆-Alkyl)carbonyloxy, (C₁-C₆-Alkoxy)-carbonyl, Phenyl, Cyano oder Nitro substituiert ist oder für (C₁-C₈-Alkyl)carbonyl stehen, welche substituiert sind durch 1-6 gleiche oder verschiedene Reste der Reihe C₁-C₆-Alkoxy, C₁-C₆-Alkylthio, (C₁-C₆-Alkyl)carbonyloxy, (C₁-C₆-Alkoxy)-carbonyl, Cyano oder Nitro oder R² und R³ zusammen mit dem durch sie eingeschlossenen Stickstoff einen 4- bis 8-gliedrigen Ring bilden,
- R⁴: für Wasserstoff, Phenyl oder C₁- bis C₈-Alkyl steht, welches gegebenenfalls substituiert ist durch 1 bis 6 Reste der Reihe Halogen, C₁-C₆-Alkoxy, C₁-C₆-Alkylthio, (C₁-C₆-Alkyl)carbonyloxy, (C₁-C₆-Alkoxy)-carbonyl, Phenyl, Cyano oder Nitro,
- Ar: für Phenyl steht, welches gegebenenfalls ein- bis fünffach gleich oder verschieden substituiert ist durch Halogen, C₁-C₈-Alkyl, C₂-C₈-Alkenyl, C₂-C₈-Alkinyl, wobei die Alkyl-, Alkenyl oder Alkinylreste gegebenenfalls substituiert sind durch 1 bis 6 Reste der Reihe Halogen, C₁-C₅-Alkoxy, welches gegebenenfalls substituiert ist durch 1 bis 6 Halogenatome, C₁-C₅-Thioalkyl, welches gegebenenfalls substituiert ist durch 1 bis 6 Reste der Reihe Halogen, (C₁-C₅-Alkyl)carbonyloxy durch C₁-C₈-Alkoxy, C₂-C₈-Alkenoxy, C₂-C₈-Alkinoxy, wobei die Alkoxy-, Alkenoxy-, Alkinoxyreste gegebenenfalls durch 1 bis 6 Halogenatome substituiert sind, durch C₁-C₈-Thioalkyl, C₂-C₈-Thioalkenyl, C₂-C₈-Thioalkinyl, wobei die Thioalkyl-, Thioalkenyl-, Thioalkinylreste gegebenenfalls durch 1 bis 6 Halogenatome substituiert sind, durch (C₁-C₈-Alkyl)carbonyloxy), welches gegebenenfalls durch 1 bis 6 Halogenatome substituiert ist, durch Amino, welches gegebenenfalls substituiert ist durch 1 bis 2 gleiche oder verschiedene Alkylreste mit 1 bis 8 Kohlenstoffatomen, welche gegebenenfalls substituiert sind durch 1 bis 6 Halogenatome, durch Nitro oder Cyano,
- Y¹: für Halogen steht und
- Y²: für Halogen oder C₁-C₆-Alkyl, welchem gegebenenfalls gleich oder verschieden substituiert ist durch 1 bis 8 Halogenatome, steht,
gefunden.

Weiterhin wurde gefunden, daß man die substituierten 2-Arylpyrrole der allgemeinen Formel (I), in welcher R¹, R², R³, R⁴, Ar, Y¹ und Y² die oben angegebene Bedeutung haben, erhält,
wenn man 2-Arylpyrrole der Formel (II) 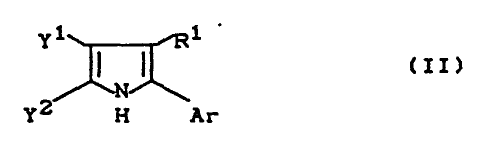 in welcher
- R¹, Ar, Y¹ und Y²: die oben angegebene Bedeutung haben,
mit Verbindungen der Formel (III) 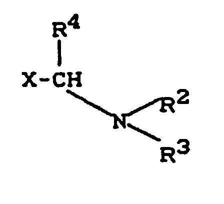 in welcher
- R², R³ und R⁴: die oben angegebene Bedeutung haben und
- X: für eine anionische Abgangsgruppe steht, gegebenenfalls in Gegenwart von Basen und/oder gegebebenenfalls in Gegenwart von Verdünnungsmitteln umsetzt.

Schließlich wurde gefunden, daß die neuen substituierten 2-Arylpyrrole der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft in den Forsten, im Vorrats- und Materialschutz sowie auf dem Hygienesektor vorkommen, geeignet sind.

Die erfindungsgemäßen neuen substituierten 2-Arylpyrrole sind durch die allgemeine Formel (I) definiert.

Bevorzugt sind substituierte 2-Arylpyrrole der allgemeinen Formel (I), in welcher
- R¹: für Cyano oder Nitro steht,
- R² und R³: unabhängig voneinander entweder für Wasserstoff, C₁-C₆-Alkyl, C₃-C₆-Alkenyl oder C₃-C₆-Alkinyl stehen, wobei die Alkyl-, Alkenyl- oder Alkinylreste gegebenenfalls durch 1 bis 5 Reste der Reihe Fluor und/oder Chlor, C₁-C₄-Alkoxy, C₁-C₄-Alkylthio, C₁-C₄-Acyloxy, (C₁-C₄-Alkoxy)-carbonyl, Phenyl, Cyano oder Nitro substituiert sind, oder für (C₁-C₆-Alkoxy)-carbonyl, (C₃-C₆-Alkenoxy)-carbonyl oder (C₃-C₆)-Alkinoxy)-carbonyl stehen, wobei der Alkoxy-, Alkenoxy- oder Alkinoxyteil gegebenenfalls durch 1 bis 5 Reste der Reihe Fluor und/oder Chlor, C₁-C₄-Alkoxy, C₁-C₄-Alkylthio, C₁-C₄-Acyloxy, (C₁-C₄-Alkoxy)-carbonyl, Phenyl, Cyano oder Nitro substituiert sind, oder für (C₁-C₆-Alkyl)carbonyl stehen, welche substituiert sind durch 1 bis 5 Reste der Reihe (C₁-C₄-Alkoxy)-carbonyl, Cyano oder Nitro oder R² und R³ zusammen mit dem durch sie eingeschlossenen Stickstoffatome einen 4 bis 6-gliedrigen Ring bilden,
- R⁴: für Wasserstoff, Phenyl, C₁- bis C₆-Alkyl steht, welches gegebenenfalls durch 1 bis 5 Reste der Reihe Fluor und/oder Chlor, C₁-C₄-Alkoxy, C₁-C₄-Alkylthio, C₁-C₄-Acyloxy, (C₁-C₄-Alkoxy)-carbonyl, Phenyl, Cyano oder Nitro substituiert ist,
- Ar: für Phenyl steht, welches gegebenenfalls ein- bis vierfach, gleich oder verschieden substituiert ist durch Fluor, Chlor oder Brom, C₁-C₆-Alkyl, C₂-C₆-Alkenyl, C₂-C₆-Alkinyl, wobei die Alkyl-, Alkenyl-, oder Alkinylreste gegebenenfalls substituiert sind durch 1 bis 5 Fluor- und/oder Chloratome, C₁-C₄-Alkoxy, welches gegebenenfalls substituiert ist durch 1 bis 5 Fluor und/oder Chloratome, C₁-C₄-Thioalkyl, welchem gegebenenfalls substituiert ist durch 1 bis 5 Fluor und/oder Chloratome, C₁-C₄-Alkoxy, welchem gegebenenfalls substituiert ist durch 1 bis 5 Fluor und/oder Chloratome, C₁-C₄-Thioalkyl, welches gegebenenfalls substituiert ist durch 1 bis 5 Fluor und/oder Chloratome, C₁-C₄-Acyloxy, durch C₁-C₆-Alkoxy, C₂-C₆-Alkenoxy, C₂-C₆-Alkinoxy, wobei die Alkoxy-, Alkenoxy-, Alkinoxyreste gegebenenfalls durch 1 bis 5 Fluor und/oder Chloratome substituiert sind, durch C₁-C₆-Thioalkyl, C₂-C₆-Thioalkenyl, Thioalkinyl, wobei die Thioalkyl-, Thioalkenyl-, Thioalkinylreste gegebenenfalls durch 1 bis 5 Fluor und/oder Chloratome substituiert sind, durch (C₁-C₆-Alkyl)carbonyloxy, welches gegebenenfalls durch 1 bis 5 Fluor und/oder Chloratome substituiert ist, durch Amino, welches gegebenenfalls substituiert ist durch 1 bis 2 gleiche oder verschiedene Alkylreste mit 1 bis 6 Kohlenstoffatomen, welche gegebenenfalls substituiert sind durch 1 bis 5 Fluor und/oder Chloratome, durch Nitro oder Cyano,
- Y¹: für Chlor oder Brom und
- Y²: für Chlor oder Brom oder C₁-C₅-Alkyl, welches gegebenenfalls gleich oder verschieden substituiert ist durch 1 bis 7 Fluor und/oder Chloratome, steht.

Verwendet man gemäß angegebenen Verfahren 4-Brom-3-Cyano-2-(4-Chlorphenyl)-5-trifluormethyl-pyrrol und N-Methyl-N-chlormethyl-carbaminsäureethylester als Ausgangsprodukte, so kann der Reaktionsverlauf durch das folgende Reaktionsschema wiedergegeben werden:

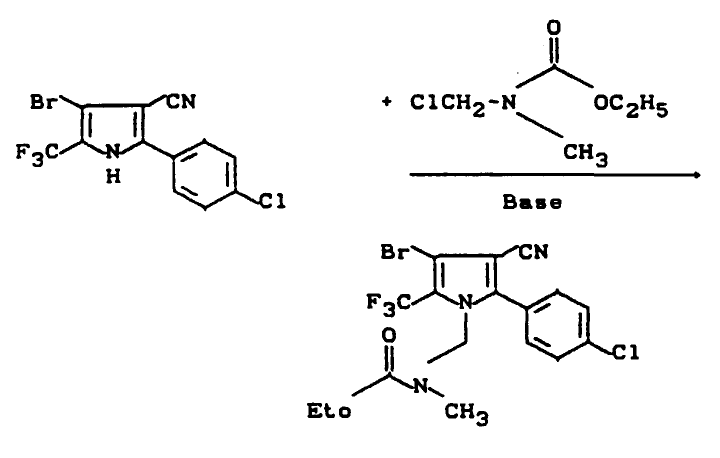

Verwendet man gemäß dem angegebenen Verfahren 2-(4-Chlorphenyl)-3-cyano-4,5-dichlorpyrrol und N-Chlormethylpyrrolidin als Ausgangsstoffe, so kann der Reaktionsverlauf durch das folgende Reaktionsschema wiedergegeben werden:

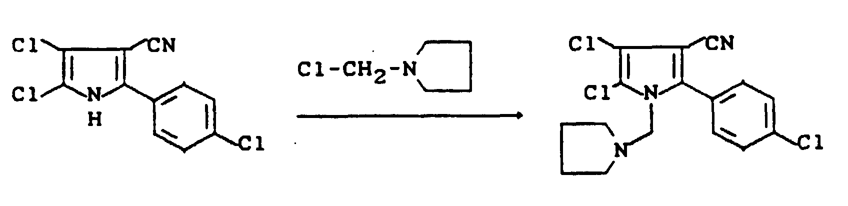

Die beim Herstellungsverfahren verwendeten Ausgangsstoffe der allgemeinen Formel (II) sind bekannt und lassen sich nach bekannten Methoden herstellen (siehe z.B. I.A. Benages et al, J. Org. Chem. 43, 4278 (1978), EP-A 0 347 480, EP-0 426 948 A1 oder US-P 5 030 735).

Die beim Herstellungsverfahren verwendeten Ausgangsstoffe der allgemeinen Formel (III) sind ebenfalls bekannt, oder lassen sich nach im Prinzip bekannten Methoden (siehe z.B. K.G. Siver et al, J. Pharm. Sci. 79, 66 (1990)) herstellen.

Das Verfahren zur Herstellung von Verbindungen der Formel (I) ist dadurch gekennzeichnet, daß man Verbindungen der Formel (II) mit Verbindungen der Formel (III), gegebenenfalls in Gegenwart von Basen und gegegebenenfalls in Gegenwart von Verdünnungsmitteln umsetzt.

Als Verdünnungsmittel kommen alle inerten organischen Lösungsmittel in Frage. Hierzu gehören vorzugsweise Kohlenwasserstoffe wie Benzol, Toluol, Xylol, ferner Ether; wie Dibutylether, tert.-Butylmethylether, Tetrahydrofuran, Dioxan, außerdem polare Lösungsmittel, wie Dimethylsulfoxid, Acetonitril, Sulfolan, Dimethylformamid und N-Methyl-pyrrolidon.

Als Basen können alle üblichen Protonenakzeptoren eingesetzt werden. Vorzugsweise verwendbar sind Alkalimetall- und Erdalkalimetall-oxide, -hydroxide und -carbanate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid und 18-Krone-6 eingesetzt werden können. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natriumethylat und Kalium-tert.-butylat einsetzbar.

Die Reaktionstemperaturen können bei der Durchführung in einem größeren Bereich variiert werden. Im allgemeinen arbeitet man bei Temperaturen zwischen -10°C und 200°C, vorzugsweise zwischen 0°C und 120°C.

Bei der Durchführung setzt man die Reaktionskomponenten der Formeln (II), die deprotonierenden Basen und die Komponenten der Formeln (III) im allgemeinen in etwa äquimolaren Mengen ein. Es ist jedoch auch möglich, die eine oder andere Komponente in einem größeren Überschuß (bis zu 3 Mol) zu verwenden.

Das Verfahren wird im allgemeinen unter Normaldruck durchgeführt, es kann auch unter Druck durchgeführt werden.

Die erfindungsgemäßen substituierten 2-Arylpyrrole (I) eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Formten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören;

Aus der Ordnung der Isopoda z.B. Oniscus asellus, Armadillidium vulgare, Porcellio scaber.
Aus der Ordnung der Diplopoda z.B. Blaniulus guttulatus.

Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.. Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectulanius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen substituierten 2-Arylpyrrole (I) wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge, Flöhe.

Sie sind gegen normalsensible und resistente Arten und Stämme, sowie gegen alle parasitierenden und nicht parasitierenden Entwicklungsstadien der Ektoparasiten wirksam.

Die erfindungsgemäßen substituierten 2-Arylpyrrole (I) zeichnen sich durch eine hohe insektizide Wirksamkeit aus. Sie lassen sich mit gutem Erfolg gegen pflanzenschädigende Insekten, wie beispielsweise gegen die Larven der Meerrettichblattkäfer (Phaedon cochleariae), gegen die grüne Pfirsichblattlaus (Myzus persicae) oder gegen die schwarze Bohnenblattlaus (Aphis fabae) einsetzen. Dabei zeigen die erfindungsgemäßen Wirkstoffe nicht nur protektive sondern auch blattsystemische und wurzelsystemische Eigenschaften.

Daneben eignen sich die erfindungsgemäßen substituierten 2-Arylpyrrole (I) auch zur Bekämpfung von Bodeninsekten und lassen sich beispielsweise zur Bekämpfung der Maden der Zwiebelfliege (Phorbia antiqua) im Boden einsetzen.

Außerdem besitzen die erfindungsgemäßen substituierten 2-Arylpyrrole (I) eine hohe Wirkung gegen Hygiene- und Vorratsschädlinge und lassen sich beispielsweise zur Bekämpfung der deutschen Schabe (Blattella germanica) einsetzen.

Die erfindungsgemäßen substituierten 2-Arylpyrrole (I) können in die üblichen Formulierungen übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der erfindungsgemäßen substituierten 2-Arylpyrrole (I) mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Träge stoffe für Granulate kommen in Frage: z.B. gebrochene und fraktionierte natürliche Gesteine wie Calcit, Marmor, Bims, Sepiolith, Dolomit sowie synthetische Granulate au anorganischen und organischen Mehlen sowie Granulate aus organischem Material wie Sägemehl, Kokosnußschalen, Maiskolben und Tabakstengel; als Emulgier und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synt hetische pulverige, körnige oder latexförmige Polymers verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen substituierten 2-Arylpyrrole (I) können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungegemäßen substituierten 2-Arylpyrrole (I) können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-%. Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäß substituierten 2-Arylpyrrole (I) eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäßen substituierten 2-Arylpyrrole (I) geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren.

Daneben ist auch eine Anwendung als Formkörper (Halsband, Ohrmarke) möglich.

Die biologische Wirksamkeit der erfindungsgemäßen Verbindungen soll anhand der Verwendungsbeispiele erläutert werden.

### Herstellungsbeispiele

### Beispiel 1

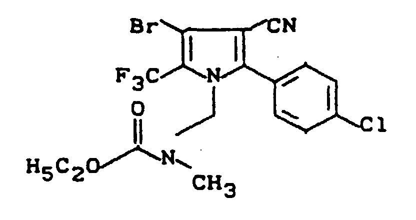

3 g (8,6 mmol) 4-Brom-3-cyano-2-(4-chlorphenyl)-5-trifluormethyl-pyrrol werden in 50 ml trockenem THF gelöst und mit 1.06 g (9.4 mmol) Kalium-tert.-butylat versetzt. Hierzu tropft man 1,34 g (9 mmol) N-Methyl-N-chlormethyl-carbaminsäureethylester, gelöst in 10 ml trockenem THF und rührt 16 h bei Raumtemperatur.

Man gießt auf Wasser und extrahiert mit CH₂Cl₂. Die org. Phasen werden vereinigt und über Na₂SO₄ getrocknet. Nach dem Einrotieren reinigt man durch Chromatographie an Kieselgel (CH₂Cl₂). Ausbeute 2,9 g (73 % d.Th.) der Verbindung obiger Formel (Physikalische Daten siehe Tabelle 1).

In analoger Weise und unter Berücksichtigung der Angaben in der Beschreibung, werden die nachfolgend in Tabelle 1 aufgeführten Substanzen der Formel (I) erhalten.

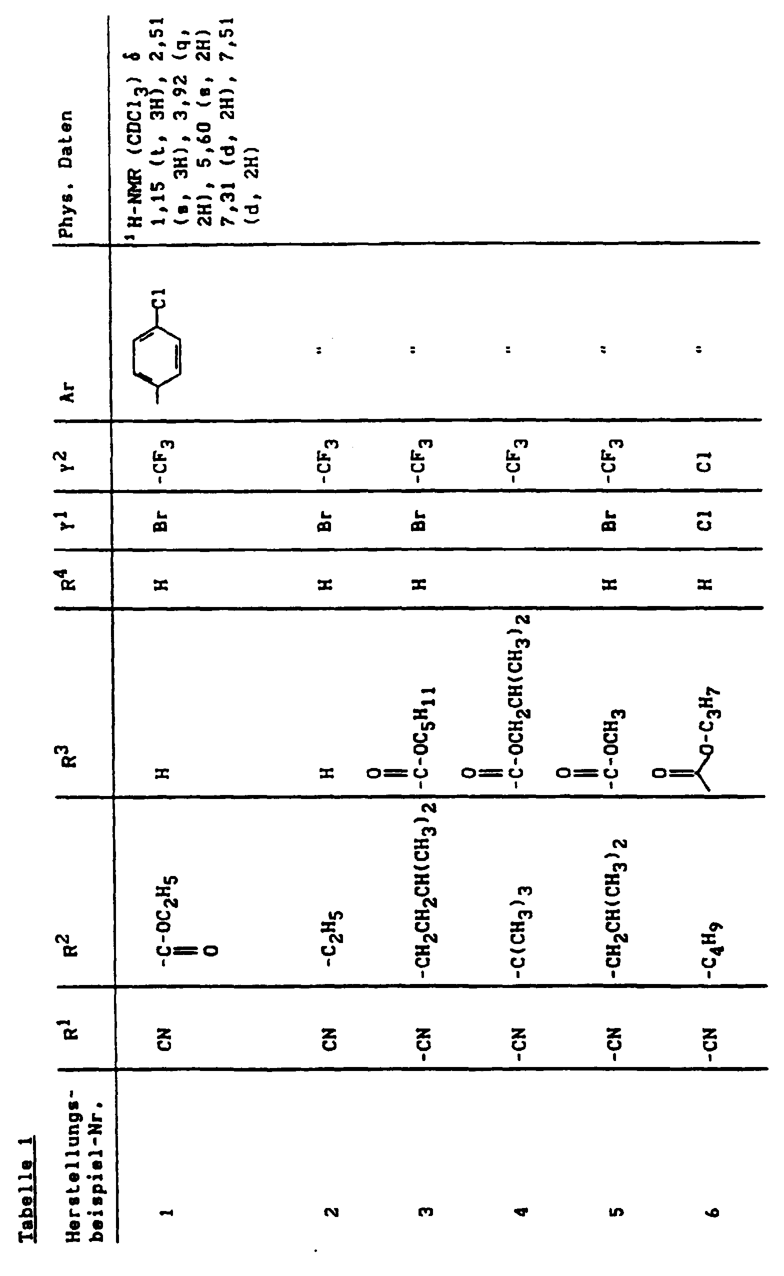

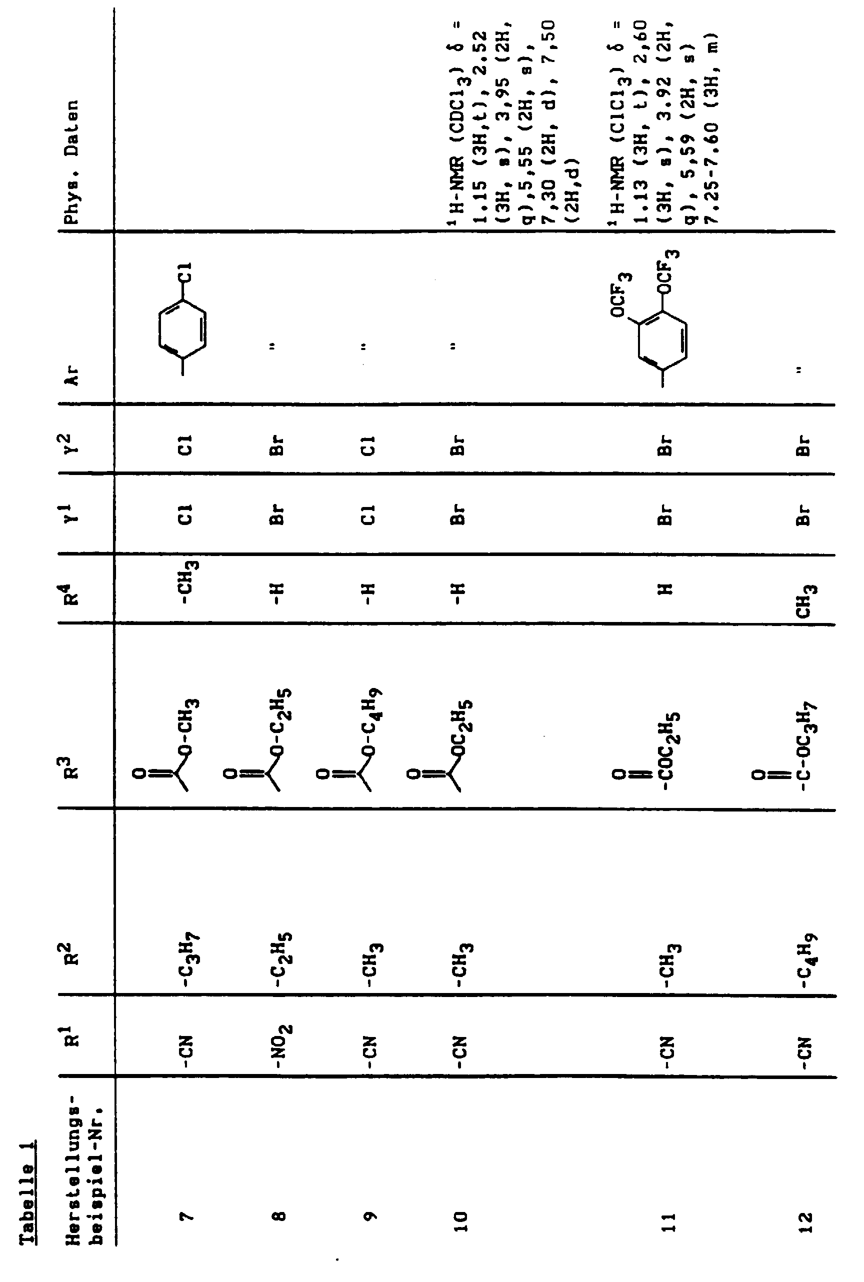

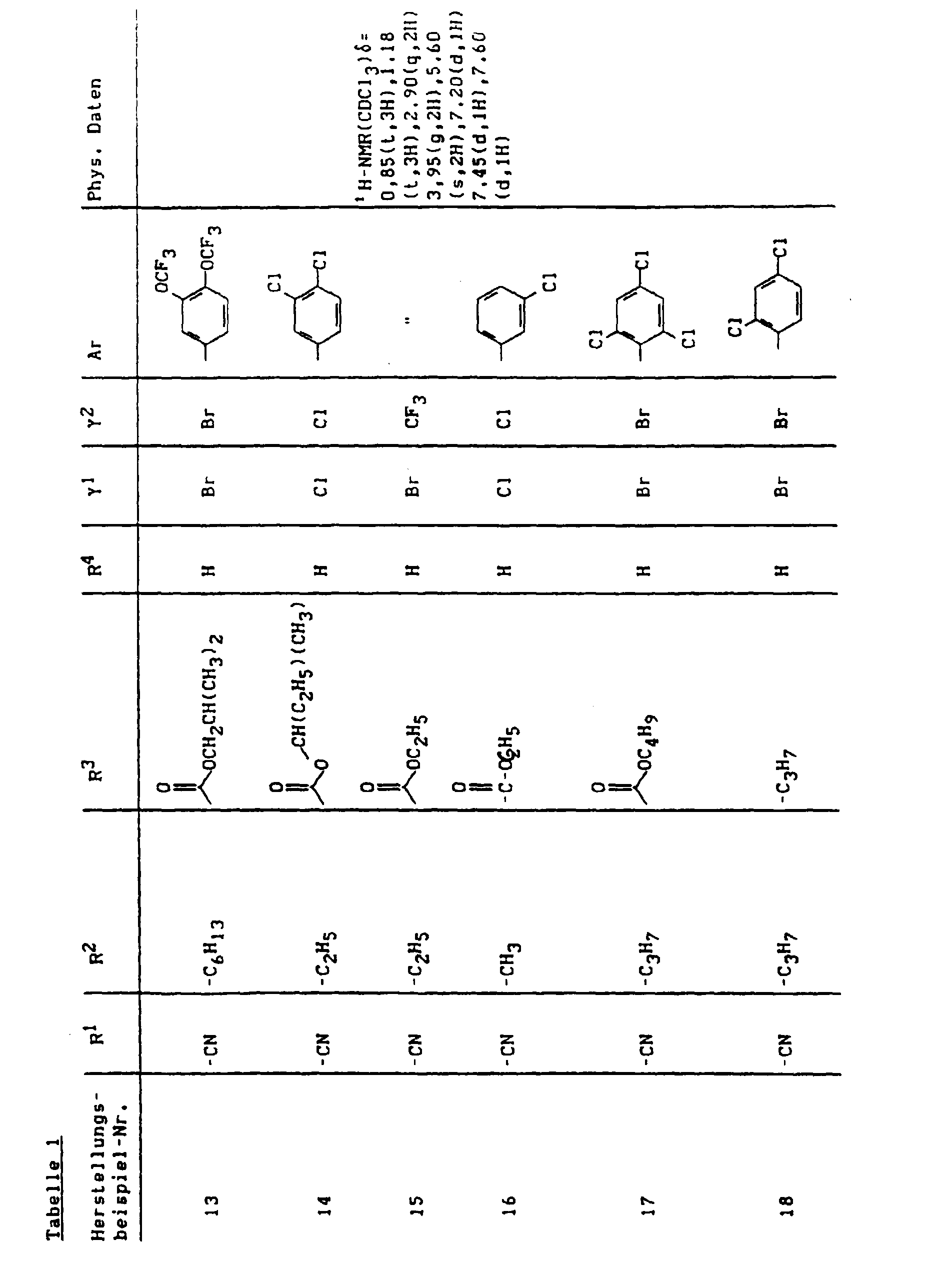

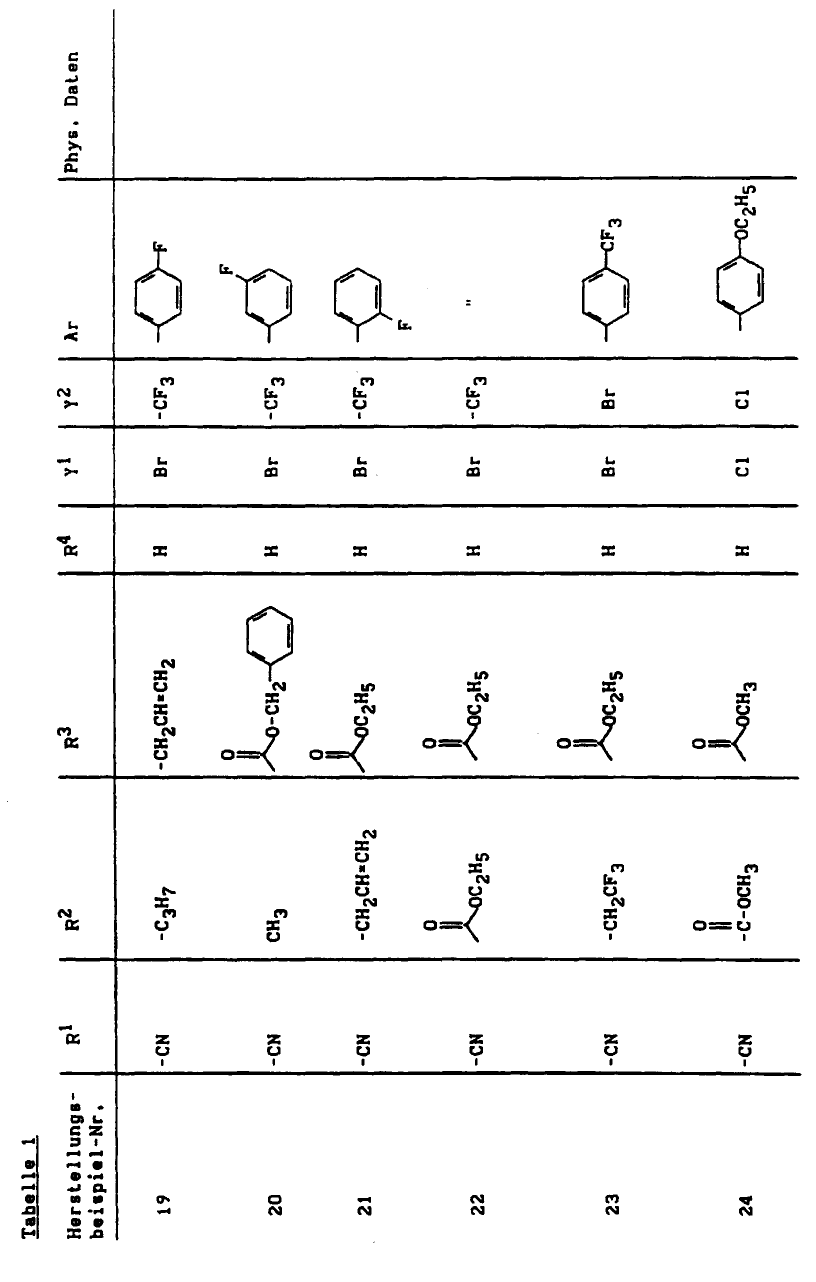

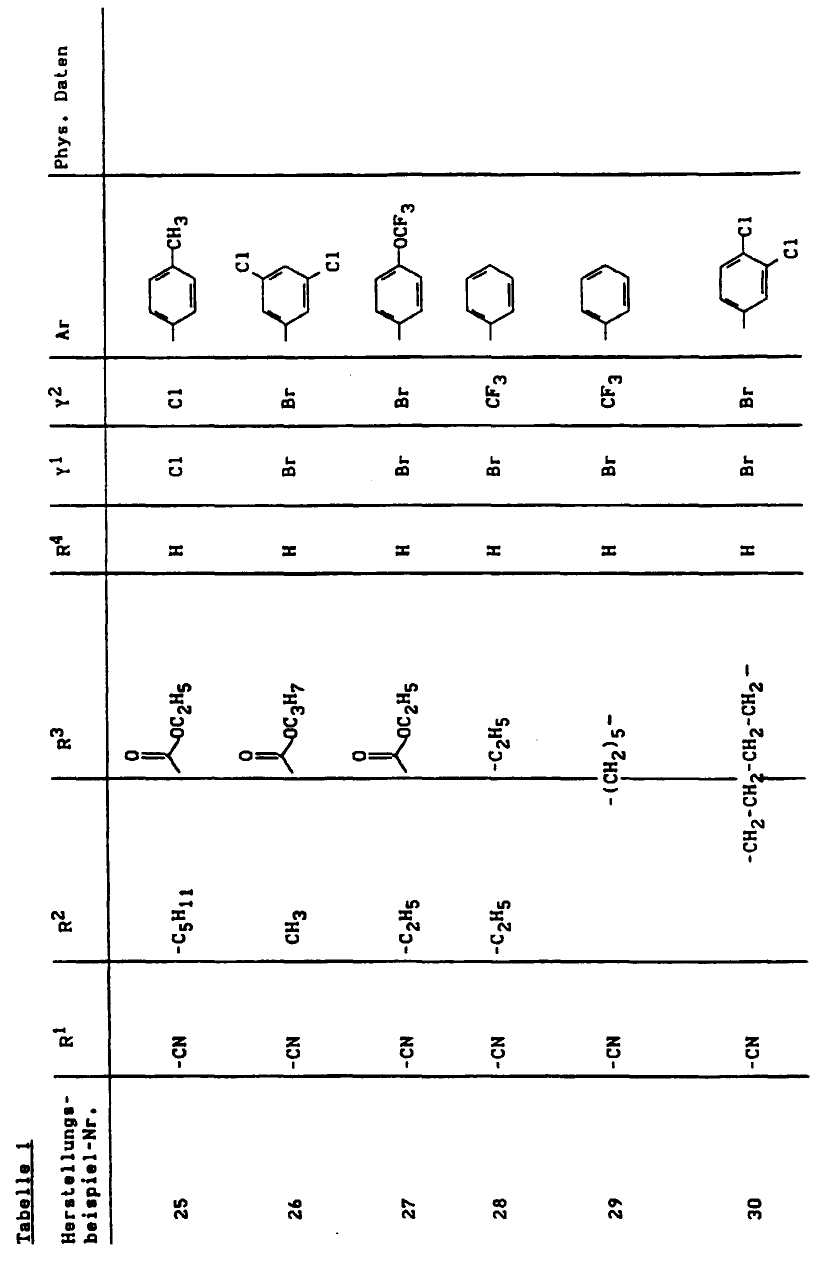

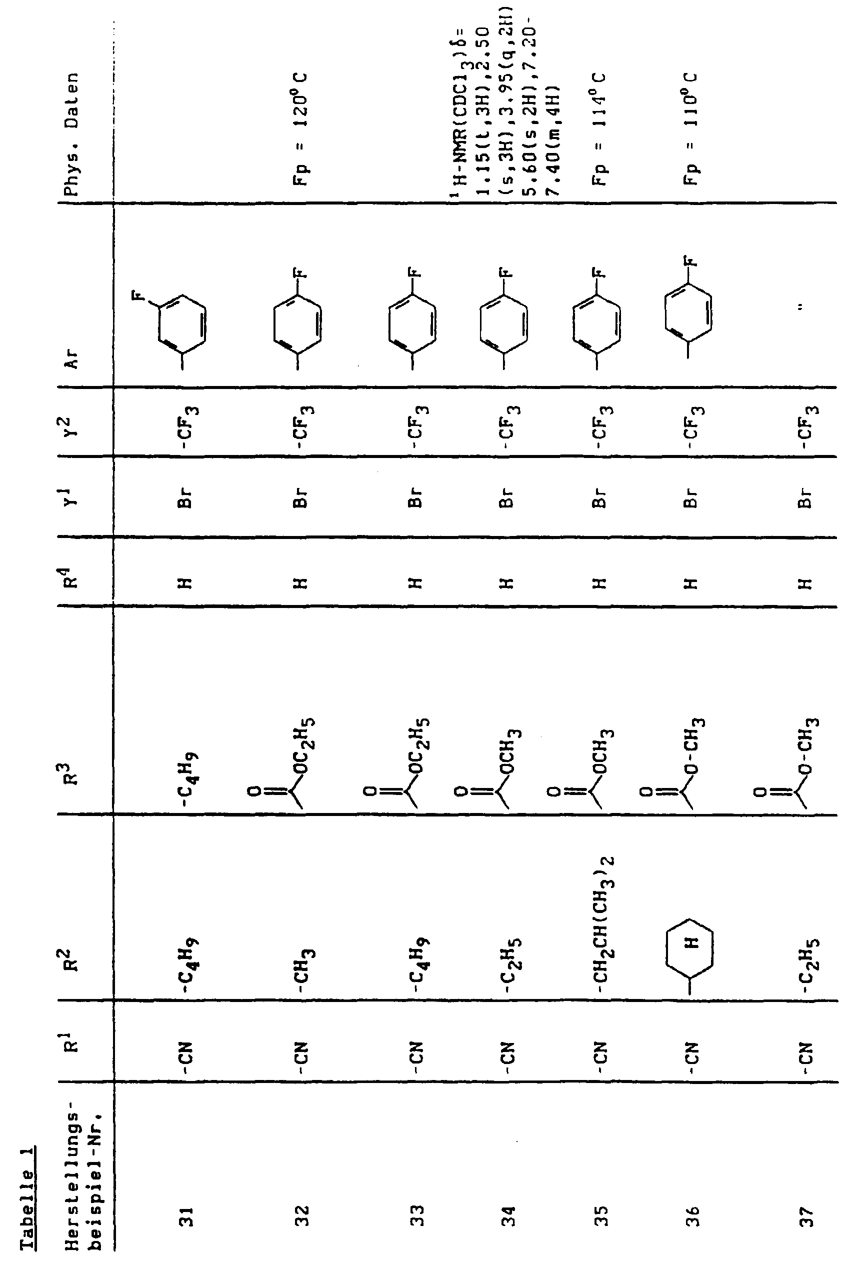

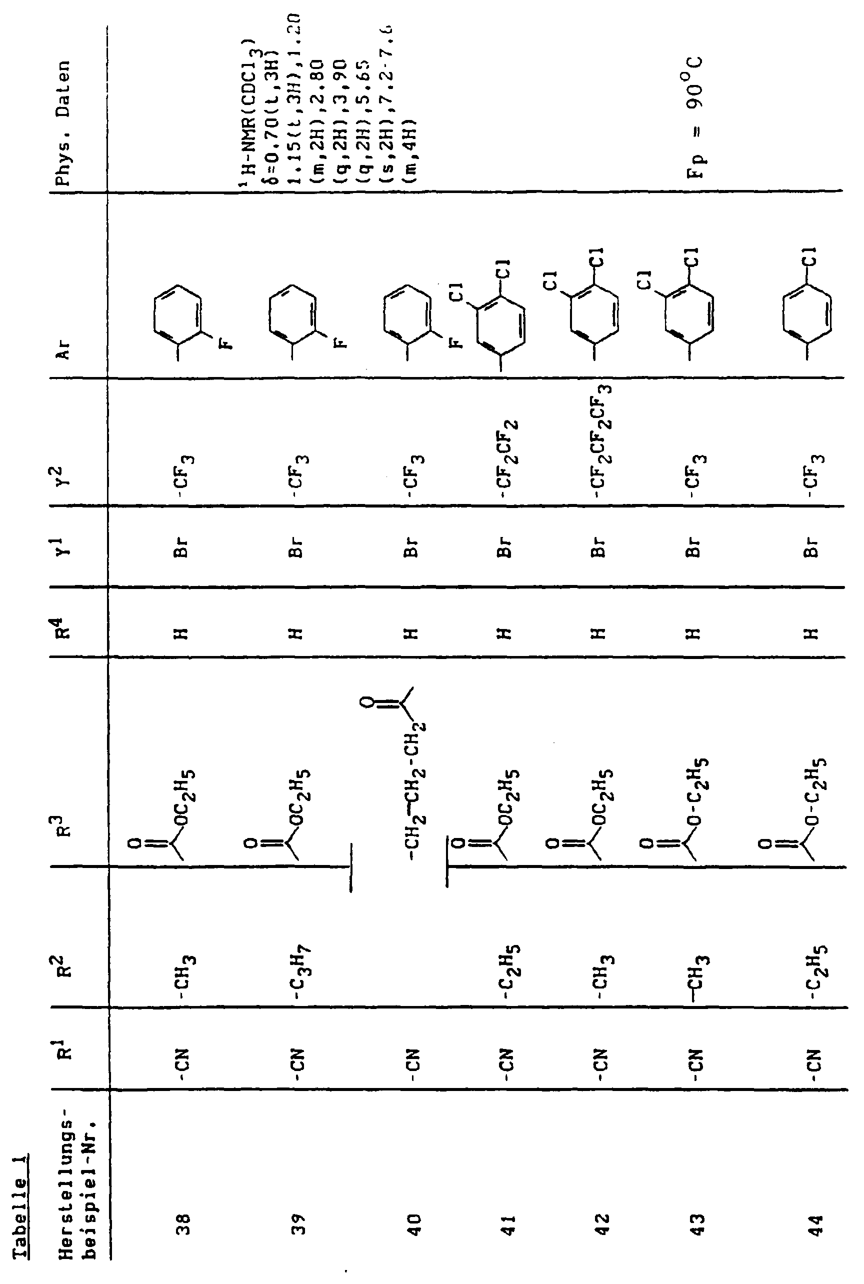

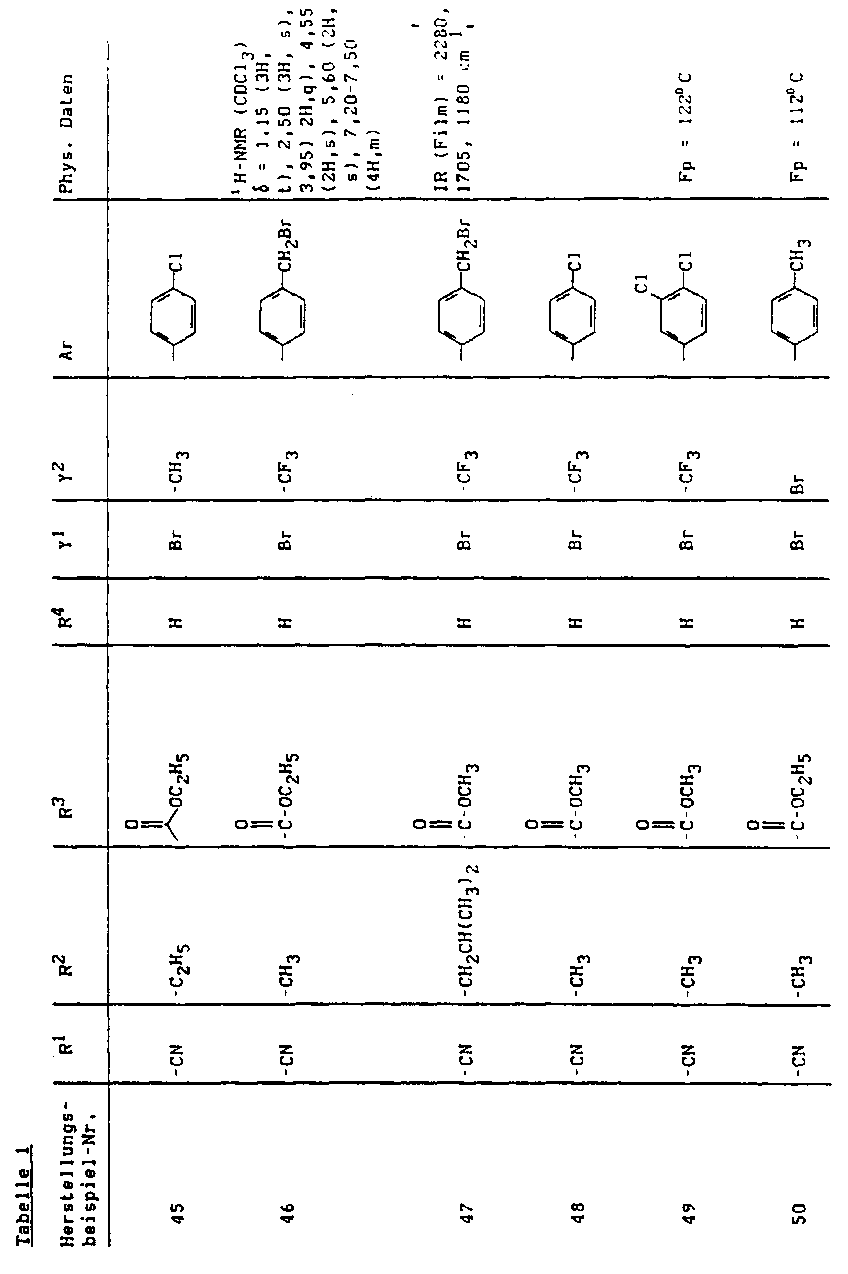

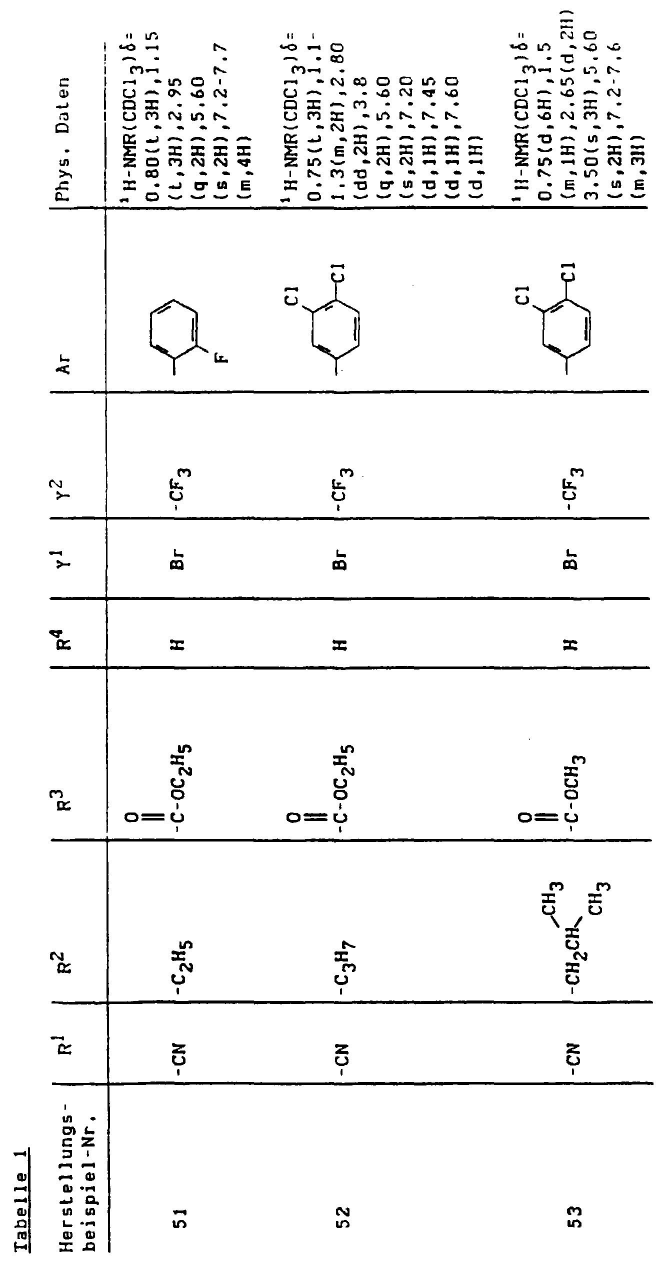

### Anwendungsbeispiele

In den folgenden Anwendungsbeispielen wurde die folgende Verbindung als Vergleichssubstanz eingesetzt:

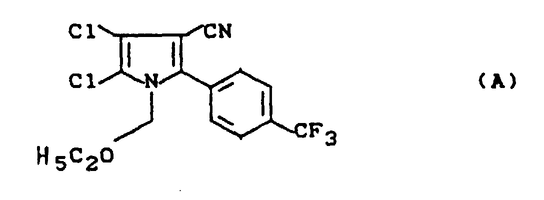

1-(Ethoxymethyl)-2-(4-trifluormethyl-phenyl)-3-cyano-4,5-dichlor-pyrrol bekannt aus EP-A 0 347 488

### Beispiel A

### Tetranychus-Test (OP-resistent)

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Bohnenpflanzen (Phaseolus vulgaris), die stark von allen Entwicklungsstadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

Bei diesem Test zeigt z.B. die folgende Verbindung der Herstellungsbeispiele überlegene Wirksamkeit gegenüber dem Stand der Technik: (1), (43), (49).

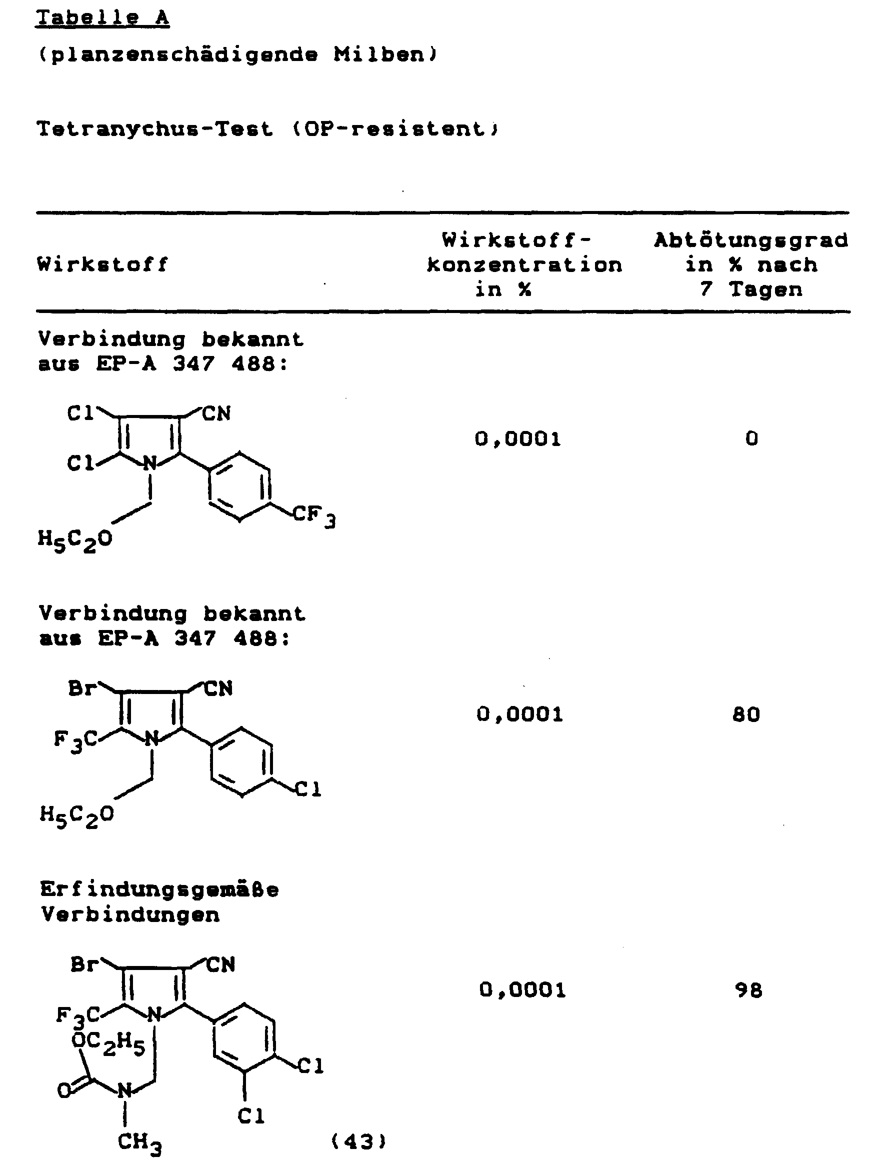

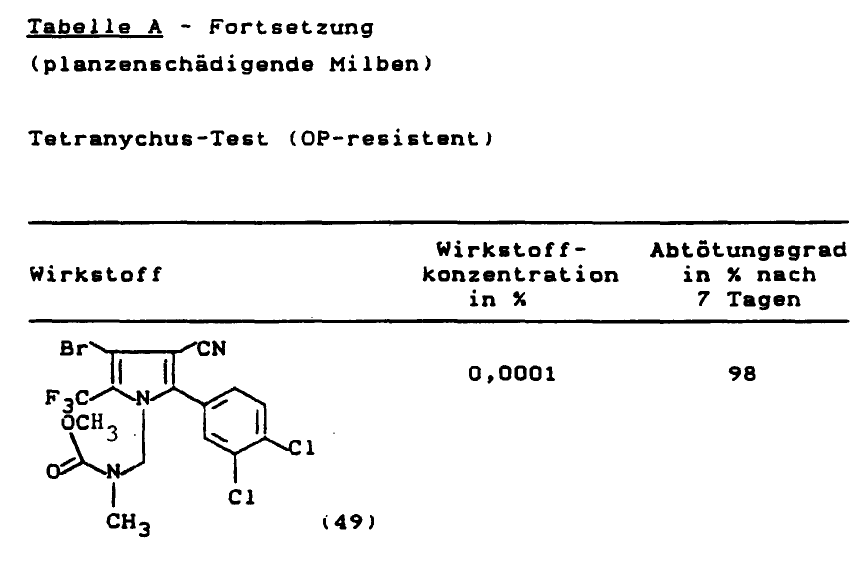

### Beispiel B

### Fliegentest

- Lösungsmittel:: 35 Gewichtsteile Ethylenglykolmonomethylether
35 Gewichtsteile Nonylphenolpolyglykolether

Zwecks Herstellung einer geeigneten Formulierung vermischt man 3 Gewichtsteile Wirkstoff mit sieben Teilen des oben angegebenen Lösungsmittels-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierscheiben (⌀ 9,5 cm) pipettiert, die sich in Petrischalen entsprechender Größe befinden. Nach Trocknung der Filterscheiben werden 25 Testtiere in die Petrischalen überführt und abgedeckt.

Nach 6 Stunden wird die Wirksamkeit der Wirkstoffzubereitung ermittelt. Die Wirksamkeit drückt man in % aus. Dabei bedeutet 100 %, daß alle Fliegen abgetötet wurden; 0 % bedeutet, daß keine Fliegen abgetötet wurden.

Bei diesem Test zeigten beispielsweise folgende erfindungsgemäße Verbindungen eine überragende biologische Wirksamkeit: Verbindungen aus Herstellungsbeispielen (15), (36).

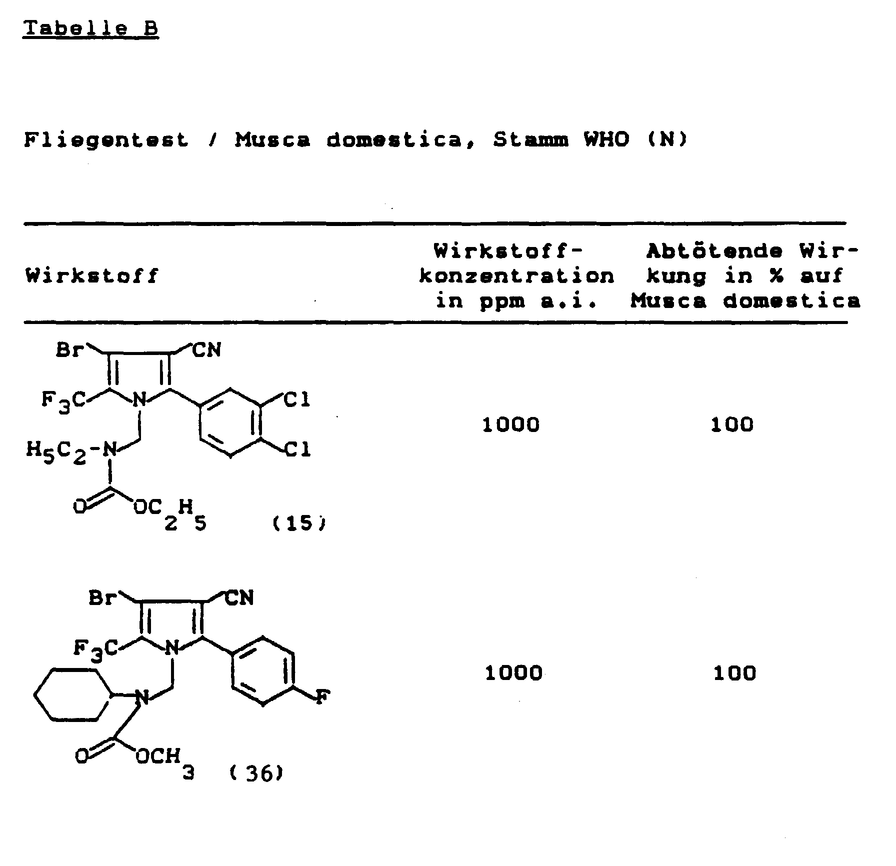

### Beispiel C

### Schabentest

- Testtiere:: Blattella germanica
- Lösungsmittel:: 35 Gewichtsteile Ethylenglykolmonomethylether
35 Gewichtsteile Nonylphenolpolyglykol ether

Zwecks Herstellung einer geeigneten Formulierung vermischt man 3 Gewichtsteile Wirkstoff mit sieben Teilen des oben angegebenen Lösungsmittels-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierscheiben (⌀ 9,5 cm) pipettiert, die sich in Petrischalen entsprechender Größe befinden. Nach Trocknung der Filterscheiben werden 5 Testtiere bei B. germanica überführt und abgedeckt.

Nach 3 Tagen wird die Wirksamkeit der Wirkstoffzubereitung bestimmt. Die Wirksamkeit drückt man in % aus. Dabei bedeutet 100 %, daß alle Schaben abgetötet wurden; 0 % bedeutet, daß keine Schaben abgetötet wurden.

Bei diesem Test zeigten beispielsweise folgende erfindungsgemäße Verbindungen eine überragende biologische Wirksamkeit: Verbindungen aus Herstellungsbeispielen (1), (15), (36), (52).

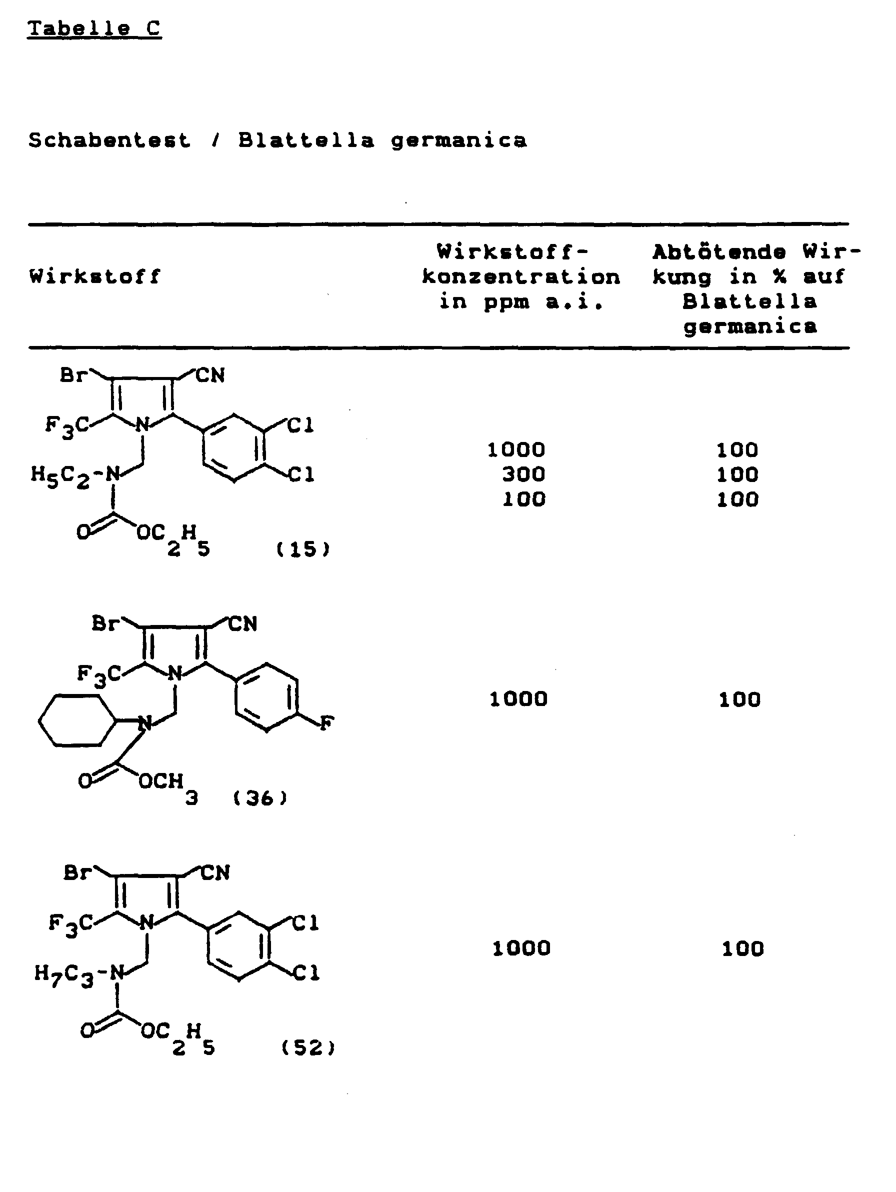

### Beispiel D

### Blowfly-Larven-Test

- Testtiere:: Lucilia cuprina-Larven
- Emulgator:: 35 Gewichtsteile Ethylenglykolmonomethylether
35 Gewichtsteile Nonylphenolpolyglykolether

Zur Herstellung einer zweckmäßigen Wirkstoffzubereitung vermischt man 3 Gewichtsteile Wirkstoff mit 7 Gewichtsteilen des oben angegebenen Gemisches und verdünnt das so erhaltene Emulsionskonzentrat mit Wasser auf die jeweils gewünschte Konzentration.

Etwa 20 Lucilia cuprina res.-Larven werden in ein Teströhrchen gebracht, welches ca. 1 cm³ Pferdefleisch und 0,5 ml der Wirkstoffzubereitung enthält. Nach 24 Stunden wird die Wirksamkeit der Wirkstoffzubereitung ermittelt. Dabei bedeutet 100 %, daß alle Blowfly-Larven abgetötet wurden; 0 % bedeutet, daß keine Blowfly-Larven abgetötet wurden.

Bei diesem Test zeigten z.B. folgende Verbindungen eine überragende biologische Wirksamkeit: Verbindungen aus Herstellungebeispielen: (15), (52).

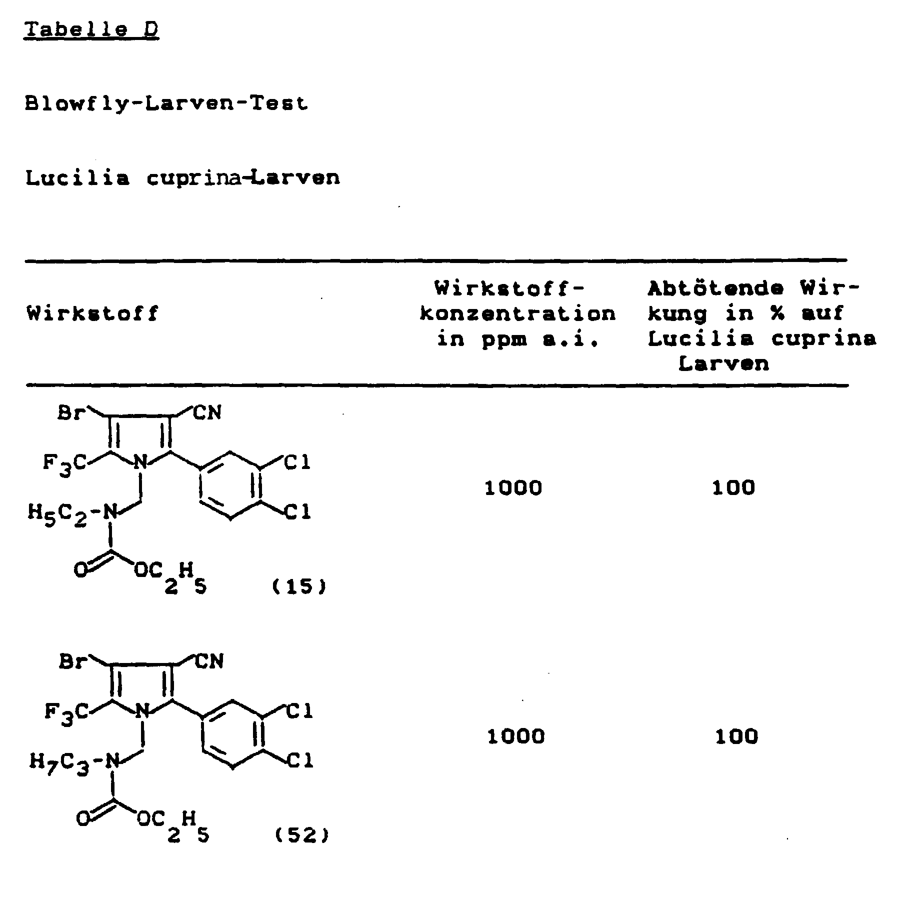

### Beispiel E

### Plutella-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentrationen.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungebeispiele überlegene Wirksamkeit gegenüber dem Stand der Technik: (1).

## Patentansprüche

1. Substituierte 2-Arylpyrrole der allgemeinen Formel (I) 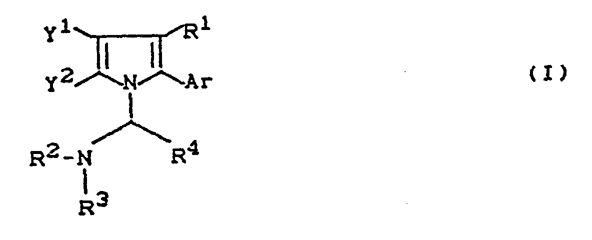 in welcher
R¹ für Cyano oder Nitro steht,
R² und R³ unabhängig voneinander entweder für Wasserstoff, C₁-C₈-Alkyl, C₃-C₈-Alkenyl oder C₃-C₈-Alkinyl stehen, wobei die Alkyl-, Alkenyl- oder Alkinylreste gegebenenfalls durch 1 bis 6 gleiche oder verschiedene Halogenatome, C₁-C₆-Alkoxy, C₁-C₆-Alkylthio, (C₁-C₆-Alkyl)carbonyloxy), (C₁-C₆-Alkoxy)-carbonyl, Phenyl, Cyano oder Nitro substituiert sind, oder für (C₁-C₈-Alkoxy)-carbonyl, (C₃-C₈-Alkenoxy)-carbonyl oder (C₃-C₈-Alkinoxy)-carbonyl stehen, wobei den Alkoxy-, Alkenoxy- oder Alkinoxyteil gegebenenfalls durch 1 bis 6 gleiche oder verschiedene Reste der Reihe Halogen, C₁-C₆-Alkoxy, C₁-C₆-Alkylthio, (C₁-C₆-Alkyl)carbonyloxy, (C₁-C₆-Alkoxy)-carbonyl, Phenyl, Cyano oder Nitro substituiert ist oder für (C₁-C₈-Alkyl)-carbonyl stehen, welche substituiert sind durch 1-6 gleiche oder verschiedene Reste der Reihe C₁-C₆-Alkoxy, C₁-C₆-Alkylthio, (C₁-C₆-Alkyl)carbonyloxy, (C₁-C₆-Alkoxy)-carbonyl, Cyano oder Nitro oder R^{²} und R³ zusammen mit dem durch sie eingeschlossenen Stickstoff einen 4- bis 8-gliedrigen Ring bilden,
R⁴ für Wasserstoff, Phenyl oder C₁- bis C₈-Alkyl steht, welches gegebenenfalls substituiert ist durch 1 bis 6 Reste der Reihe Halogen, C₁-C₆-Alkoxy, C₁-C₆-Alkylthio, (C₁-C₆-Alkyl)carbonyloxy, (C₁-C₆-Alkoxy)-carbonyl, Phenyl, Cyano oder Nitro,
Ar für Phenyl steht, welches gegebenenfalls ein-bis fünffach gleich oder verschieden substituiert ist durch Halogen, C₁-C₈-Alkyl, C₂-C₈-Alkenyl, C₂-C₈-Alkinyl, wobei die Alkyl-, Alkenyl oder Alkinylreste gegebenenfalls substituiert sind durch 1 bis 6 Reste der Reihe Halogen, C₁-C₅-Alkoxy, welches gegebenenfalls substituiert ist durch 1 bis 6 Halogenatome, C₁-C₅-Thioalkyl, welches gegebenenfalls substituiert ist durch 1 bis 6 Reste der Reihe Halogen, (C₁-C₅-Alkyl)-carbonyloxy durch C₁-C₈-Alkoxy, C₂-C₈-Alkenoxy, C₂-C₈-Alkinoxy, wobei die Alkoxy-, Alkenoxy-, Alkinoxyreste gegebenenfalls durch 1 bis 6 Halogenatome substituiert sind, durch C₁-C₈-Thioalkyl, C₂-C₈-Thioalkenyl, C₂-C₈-Thioalkinyl, wobei die Thioalkyl-, Thioalkenyl-, Thioalkinylreste gegebenenfalls durch 1 bis 6 Halogenatome substituiert sind, durch (C₁-C₈-Alkyl)carbonyloxy), welches gegebenenfalls durch 1 bis 6 Halogenatome substituiert ist, durch Amino, welches gegebenenfalls substituiert ist durch 1 bis 2 gleiche oder verschiedene Alkylreste mit 1 bis 8 Kohlenstoffatomen, welche gegebenenfalls substituiert sind durch 1 bis 6 Halogenatome, durch Nitro oder Cyano,
Y¹ für Halogen steht und
Y² für Halogen oder C₁-C₆-Alkyl, welches gegebenenfalls gleich oder verschieden substituiert ist durch 1 bis 8 Halogenatome, steht.

2. 2-Arylpyrrole der allgemeinen Formel (I) gemäß Anspruch 1, in welcher
R¹ für Cyano oder Nitro steht,
R² und R³ unabhängig voneinander entweder für Wasserstoff, C₁-C₆-Alkyl, C₃-C₆-Alkenyl oder C₃-C₆-Alkinyl stehen, wobei die Alkyl-, Alkenyl-oder Alkinylreste gegebenenfalls durch 1 bis 5 Reste der Reihe Fluor und/oder Chlor, C₁-C₄-Alkoxy, C₁-C₄-Alkylthio, C₁-C₄-Acyloxy, (C₁-C₄-Alkoxy)-carbonyl, Phenyl, Cyano oder Nitro substituiert sind, oder für (C₁-C₆-Alkoxy)-carbonyl, (C₃-C₆-Alkenoxy)-carbonyl oder (C₃-C₆-Alkinoxy)-carbonyl stehen, wobei der Alkoxy-, Alkenoxy- oder Alkinoxyteil gegebenenfalls durch 1 bis 5 Reste der Reihe Fluor und/oder Chlor, C₁-C₄-Alkoxy, C₁-C₄-Alkylthio, C₁-C₄-Acyloxy, (C₁-C₄-Alkoxy)-carbonyl, Phenyl, Cyano oder Nitro substituiert sind, oder für (C₁-C₆-Alkyl)-carbonyl stehen, welche substituiert sind durch 1 bis 5 Reste der Reihe C₁-C₄-Alkoxy)-carbonyl, Cyano oder Nitro oder R² und R³ zusammen mit dem durch sie eingeschlossenen Stickstoffatome einen 4 bis 6-gliedrigen Ring bilden,
R⁴ für Wasserstoff, Phenyl, C₁- bis C₆-Alkyl steht, welches gegebenenfalls durch 1 bis 5 Reste der Reihe Fluor und/oder Chlor, C₁-C₄-Alkoxy, C₁-C₄-Alkylthio, C₁-C₄-Acyloxy, (C₁-C₄-Alkoxy)-carbonyl, Phenyl, Cyano oder Nitro substituiert ist,
Ar für Phenyl steht, welches gegebenenfalls ein- bis vierfach, gleich oder verschieden substituiert ist durch Fluor, Chlor oder Brom, C₁-C₆-Alkyl, C₂-C₆-Alkenyl, C₂-C₆-Alkinyl, wobei die Alkyl-, Alkenyl-, oder Alkinylreste gegebenenfalls substituiert sind durch 1 bis 5 Fluor- und/oder Chloratome, C₁-C₄-Alkoxy, welches gegebenenfalls substituiert ist durch 1 bis 5 Fluor und/oder Chloratome, C₁-C₄-Thioalkyl, welches gegebenenfalls substituiert ist durch 1 bis 5 Fluor und/oder Chloratome, C₁-C₄-Alkoxy, welches gegebenenfalls substituiert ist durch 1 bis 5 Fluor und/oder Chloratome, C₁-C₄-Thioalkyl, welches gegebenenfalls substituiert ist durch 1 bis 5 Fluor und/oder Chloratome, C₁-C₄-Acyloxy, durch C₁-C₆-Alkoxy, C₂-C₆-Alkenoxy, C₂-C₆-Alkinoxy, wobei die Alkoxy-, Alkenoxy-, Alkinoxyreste gegebenenfalls durch 1 bis 5 Fluor und/oder Chloratome substituiert sind, durch C₁-C₆-Thioalkyl, C₂-C₆-Thioalkenyl, Thioalkinyl, wobei die Thioalkyl-, Thioalkenyl-, Thioalkinylreste gegebenenfalls durch 1 bis 5 Fluor und/oder Chloratome substituiert sind, durch (C₁-C₆-Alkyl)-carbonyloxy, welches gegebenenfalls durch 1 bis 5 Fluor und/oder Chloratome substituiert ist, durch Amino, welches gegebenenfalls substituiert ist durch 1 bis 2 gleiche oder verschiedene Alkylreste mit 1 bis 6 Kohlenstoffatomen, welche gegebenenfalls substituiert sind durch 1 bis 5 Fluor und/oder Chloratome, durch Nitro oder Cyano,
Y¹ für Chlor oder Brom und
Y² für Chlor oder Brom oder C₁-C₅-Alkyl, welches gegebenenfalls gleich oder verschieden substituiert ist durch 1 bis 7 Fluor und/oder Chloratome, steht.

3. Verfahren zur Herstellung von substituierten 2-Arylpyrrolen der allgemeinen Formel (I), in welcher
R¹, R², R³, R⁴, Ar, Y¹ und Y² die in Anspruch 1 angegebene Bedeutung haben, dadurch gekennzeichnet, daß man 2-Arylpyrrole der Formel (II) 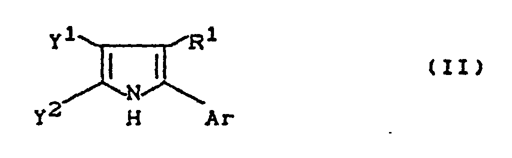 in welcher
R¹, Ar, Y¹ und Y² die oben angegebene Bedeutung haben,
mit Verbindungen der Formel (III) 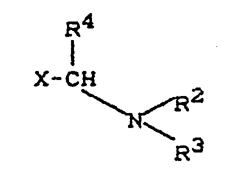 in welcher
R², R³ und R⁴ die oben angegebene Bedeutung haben und
X für eine anionische Abgangsgruppe steht,
gegebenenfalls in Gegenwart von Basen und/oder gegebenenfalls in Gegenwart von Verdünnungsmitteln umsetzt.

4. Schädlingsbekämpfungsmittel, gekennzeichnet durch einen Gehalt an mindestens einem substituierten 2-Arylpyrrol der Formel (I).

5. Verfahren zur Bekämpfung von tierischen Schädlingen, dadurch gekennzeichnet, daß man substituierte 2-Arylpyrrole der Formel (I) auf tierische Schädlinge und/oder ihren Lebensraum einwirken läßt.

6. Verwendung von substituierten 2-Arylpyrrolen der Formel (I) zur Bekämpfung von tierischen Schädlingen.

7. Verfahren zur Herstellung von Schädlingsbekämfungsmitteln, dadurch gekennzeichnet, daß man substituierte 2-Arylpyrrole der Formel (I) mit Streckmitteln und/oder oberflächenaktiven Mitteln vermischt.

## Claims

1. Substituted 2-arylpyrroles of the general formula (I) 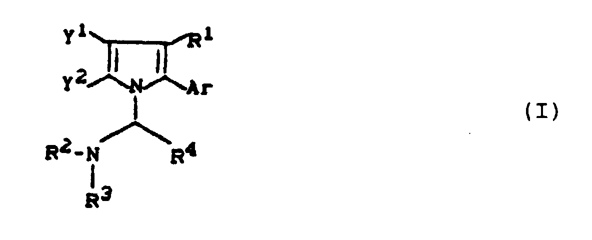 in which
R¹ represents cyano or nitro,
R² and R³, independently of each other, either represent hydrogen, C₁-C₈-alkyl, C₃-C₈-alkenyl or C₃-C₈-alkinyl, where the alkyl, alkenyl or alkinyl radicals are optionally substituted by 1 to 6 identical or different halogen atoms, C₁-C₆-alkoxy, C₁-C₆-alkylthio, (C₁-C₆-alkyl)carbonyloxy, (C₁-C₆-alkoxy)-carbonyl, phenyl, cyano or nitro, or represent (C₁-C₈-alkoxy)-carbonyl, (C₃-C₈-alkenoxy)-carbonyl or (C₃-C₈-alkinoxy)-carbonyl, where the alkoxy, alkenoxy or alkinoxy moiety is in each case optionally substituted by 1 to 6 identical or different radicals from the series halogen, C₁-C₆-alkoxy, C₁-C₆-alkylthio, (C₁-C₆-alkyl)carbonyloxy, (C₁-C₆-alkoxy)-carbonyl, phenyl, cyano or nitro, or represent (C₁-C₈-alkyl)carbonyl, which is substituted by 1-6 identical or different radicals from the series C₂-C₆-alkoxy, C₁-C₆-alkylthio, (C₁-C₆-alkyl)carbonyloxy, (C₁-C₆-alkoxy)-carbonyl, cyano or nitro, or R² and R³, together with the nitrogen enclosed by them, form a 4- to 8-membered ring,
R⁴ represents hydrogen, phenyl or C₁- to C₈-alkyl, which is optionally substituted by 1 to 6 radicals from the series halogen, C₁-C₆-alkoxy, C₁-C₆-alkylthio, (C₁-C₆-alkyl)carbonyloxy, (C₁-C₆-alkoxy)-carbonyl, phenyl, cyano or nitro,
Ar represents phenyl, which is optionally substituted identically or differently once to five times by halogen, C₁-C₈-alkyl, C₂-C₈-alkenyl or C₂-C₈-alkinyl, where the alkyl, alkenyl or alkinyl radicals are optionally substituted by 1 to 6 radicals from the series halogen, C₁-C₅-alkoxy, which is optionally substituted by 1 to 6 halogen atoms, or C₁-C₅-alkylthio, which is optionally substituted by 1 to 6 radicals from the series halogen or (C₁-C₅-alkyl)-carbonyloxy, by C₁-C₈-alkoxy, C₂-C₈-alkenoxy or C₂-C₈-alkinoxy, where the alkoxy, alkenoxy or alkinoxy radicals are optionally substituted by 1 to 6 halogen atoms, by C₁-C₈-alkylthio, C₂-C₈-alkenylthio or C₂-C₈-alkinylthio, where the alkylthio, alkenylthio or alkinylthio radicals are optionally substituted by 1 to 6 halogen atoms, by (C₁-C₈-alkyl)carbonyloxy, which is optionally substituted by 1 to 6 halogen atoms, by amino, which is optionally substituted by 1 to 2 identical or different alkyl radicals having 1 to 8 carbon atoms, which are optionally substituted by 1 to 6 halogen atoms, by nitro or cyano,
Y¹ represents halogen and
Y² represents halogen or C₁-C₆-alkyl, which is optionally substituted identically or differently by 1 to 8 halogen atoms.

2. 2-Arylpyrroles of the general formula (I) according to Claim 1, in which
R¹ represents cyano or nitro,
R² and R³, independently of each other, either represent hydrogen, C₁-C₆-alkyl, C₃-C₆-alkenyl or C₃-C₆-alkinyl, where the alkyl, alkenyl or alkinyl radicals are optionally substituted by 1 to 5 radicals from the series fluorine and/or chlorine, C₁-C₄-alkoxy, C₁-C₄-alkylthio, C₁-C₄-acyloxy, (C₁-C₄-alkoxy)-carbonyl, phenyl, cyano or nitro, or represent (C₁-C₆-alkoxy)-carbonyl, (C₃-C₆-alkenoxy)-carbonyl or (C₃-C₆-alkinoxy)-carbonyl, where the alkoxy, alkenoxy or alkinoxy moiety is optionally substituted by 1 to 5 radicals from the series fluorine and/or chlorine, C₁-C₄-alkoxy, C₁-C₄-alkylthio, C₁-C₄-acyloxy, (C₁-C₄-alkoxy)-carbonyl, phenyl, cyano or nitro, or represent (C₁-C₆-alkyl)carbonyl, which is substituted by 1 to 5 radicals from the series (C₁-C₄-alkoxy)-carbonyl, phenyl, cyano or nitro, or R² and R³, together with the nitrogen atom enclosed by them, form a 4- to 6-membered ring,
R⁴ represents hydrogen, phenyl or C₁- to C₆-alkyl, which is optionally substituted by 1 to 5 radicals from the series fluorine and/or chlorine, C₁-C₄-alkoxy, C₁-C₄-alkylthio, C₁-C₄-acyloxy, (C₁-C₄-alkoxy)-carbonyl, phenyl, cyano or nitro,
Ar represents phenyl, which is optionally substituted identically or differently once to four times by fluorine, chlorine or bromine, C₁-C₆-alkyl, C₂-C₆-alkenyl or C₂-C₆-alkinyl, where the alkyl, alkenyl or alkinyl radicals are optionally substituted by 1 to 5 fluorine and/or chlorine atoms, C₁-C₄-alkoxy, which is optionally substituted by 1 to 5 fluorine and/or chlorine atoms, or C₁-C₄-alkylthio, which is optionally substituted by 1 to 5 fluorine and/or chlorine atoms or C₁-C₄-acyloxy, by C₁-C₆-alkoxy, C₂-C₆-alkenoxy or C₂-C₆-alkinoxy, where the alkoxy, alkenoxy or alkinoxy radicals are optionally substituted by 1 to 5 fluorine and/or chlorine atoms, by C₁-C₆-alkylthio, C₂-C₆-alkenylthio or - alkinylthio, where the alkylthio, alkenylthio or alkinylthio radicals are optionally substituted by 1 to 5 fluorine and/or chlorine atoms, by (C₁-C₆-alkyl)carbonyloxy, which is optionally substituted by 1 to 5 fluorine and/or chlorine atoms, by amino, which is optionally substituted by 1 to 2 identical or different alkyl radicals having 1 to 6 carbon atoms, which are optionally substituted by 1 to 5 fluorine and/or chlorine atoms, by nitro or cyano,
Y¹ represents chlorine or bromine and
Y² represents chlorine or bromine or C₁-C₅-alkyl, which is optionally substituted identically or differently by 1 to 7 fluorine and/or chlorine atoms.

3. Process for preparing substituted 2-arylpyrroles of the general formula (I), in which
R¹, R², R³, R⁴, Ar, Y¹ and Y² have the meaning given in Claim 1, characterised in that 2-arylpyrroles of the formula (II) 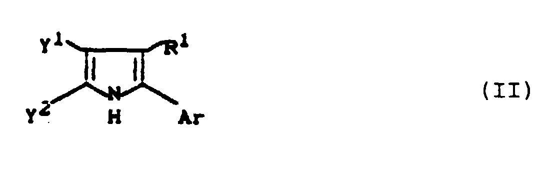 in which
R¹, Ar, Y¹ and Y² have the abovementioned meaning, are reacted with compounds of the formula (III) 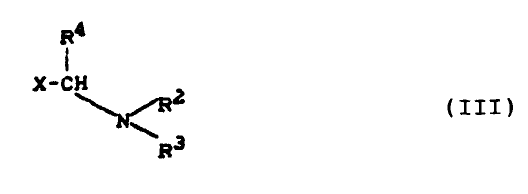 in which
R², R³ and R⁴ have the abovementioned meaning and
X represents an anionic leaving group,
optionally in the presence of bases and/or optionally in the presence of diluents.

4. Agent for combating pests, characterised by a content of at least one substituted 2-arylpyrrole of the formula (I).

5. Process for combating animal pests, characterised in that substituted 2-arylpyrroles of the formula (I) are allowed to act on animal pests and/or their habitat.

6. Use of substituted 2-arylpyrroles of the formula (I) for combating animal pests.

7. Process for preparing agents for combating pests, characterised in that substituted 2-arylpyrroles of the formula (I) are mixed with extenders and/or surface-active agents.

## Revendications

1. 2-arylpyrroles substitués de formule générale (I) 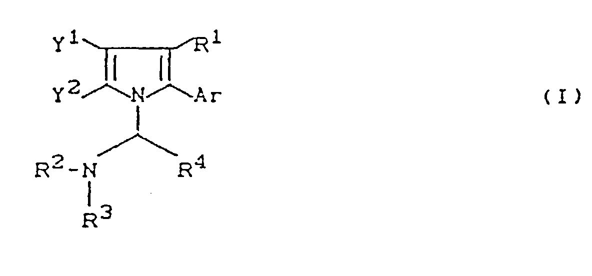 dans laquelle
R¹ est un groupe cyano ou nitro,
R² et R³ représentent, indépendamment l'un de l'autre, l'hydrogène, un reste alkyle en C₁ à C₈, alcényle en C₃ à C₈ ou un reste alcynyle en C₃ à C₈, les restes alkyle, alcényle ou alcynyle étant éventuellement substitués par 1 à 6 atomes d'halogènes, groupes alkoxy en C₁ à C₆, alkylthio en C₁ à C₆, (alkyle en C₁ à C₆)carbonyloxy, (alkoxy en C₁ à C₆)carbonyle, phényle, cyano ou nitro identiques ou différents, ou un reste (alkoxy en C₁ à C₈)-carbonyle, (alcénoxy en C₃ à C₈)-carbonyle ou (alcynoxy en C₃ à C₈)-carbonyle, la partie alkoxy, alcénoxy ou alcynoxy étant substituée le cas échéant par 1 à 6 restes, identiques ou différents, de la série halogène, alkoxy en C₁ à C₆, alkylthio en C₁ à C₆, (alkyle en C₁ à C₆)carbonyloxy, (alkoxy en C₁ à C₆)-carbonyle, phényle, cyano ou nitro, ou des restes (alkyle en C₁ à C₈)-carbonyle qui sont substitués par 1 à 6 restes identiques ou différents de la série alkoxy en C₁ à C₆, alkylthio en C₁ à C₆, (alkyle en C₁ à C₆)carbonyloxy, (alkoxy en C₁ à C₆)-carbonyle, cyano ou nitro, ou bien R² et R³ forment conjointement avec l'azote auquel ils sont liés un noyau de 4 à 8 chaînons,
R⁴ représente l'hydrogène, un groupe phényle ou un groupe alkyle en C₁ à C₈ qui est substitué le cas échéant par 1 à 6 restes de la série halogène, alkoxy en C₁ à C₆, alkylthio en C₁ à C₆, (alkyle en C₁ à C₆)carbonyloxy, (alkoxy en C₁ à C₆)-carbonyle, phényle, cyano ou nitro,
Ar représente un groupe phényle qui est substitué le cas échéant 1 à 5 fois identiques ou différentes par un halogène, un reste alkyle en C₁ à C₈, alcényle en C₂ à C₈, alcynyle en C₂ à C₈, les restes alkyle, alcényle ou alcynyle étant substitués le cas échéant par 1 à 6 restes de la série halogène, alkoxy en C₁ à C₅, qui est substitué le cas échéant par 1 à 6 atomes d'halogènes, un reste thioalkyle en C₁ à C₅ qui est substitué le cas échéant par 1 à 6 restes de la série halogène, (alkyle en C₁ à C₅)-carbonyloxy, par un reste alkoxy en C₁ à C₈, alcénoxy en C₂ à C₈, alcynoxy en C₂ à C₈, les restes alkoxy, alcénoxy, alcynoxy étant substitués le cas échéant par 1 à 6 atomes d'halogènes, par un reste thioalkyle en C₁ à C₈, thioalcényle en C₂ à C₈, thioalcynyle en C₂ à C₈, les restes thioalkyle, thioalcényle, thioalcynyle étant substitués le cas échéant par 1 à 6 atomes d'halogènes, par un reste (alkyle en C₁ à C₈)carbonyloxy, qui est substitué le cas échéant par 1 à 6 atomes d'halogènes, par un reste amino qui est substitué le cas échéant par 1 à 2 restes alkyle identiques ou différents ayant 1 à 8 atomes de carbone, qui sont éventuellement substitués par 1 à 6 atomes d'halogènes, par un reste nitro ou cyano,
Y¹ est un halogène et
Y² est un halogène ou un reste alkyle en C₁ à C₆ qui est substitué le cas échéant par 1 à 8 atomes d'halogènes identiques ou différents.

2. 2-arylpyrroles de formule générale (I) suivant la revendication 1, dans laquelle
R¹ est un groupe cyano ou nitro,
R² et R³ représentent, indépendamment l'un de l'autre, l'hydrogène, un reste alkyle en C₁ à C₆, alcényle en C₃ à C₆ ou alcynyle en C₃ à C₆, les restes alkyle, alcényle ou alcynyle étant substitués le cas échéant par 1 à 5 restes de la série fluor et/ou chlore, alkoxy en C₁ à C₄, alkylthio en C₁ à C₄, acyloxy en C₁ à C₄, (alkoxy en C₁ à C₄)-carbonyle, phényle, cyano ou nitro, ou un reste (alkoxy en C₁ à C₆)-carbonyle, (alcénoxy en C₃ à C₆)-carbonyle ou (alcynoxy en C₃ à C₆)-carbonyle, la partie alkoxy, alcénoxy ou alcynoxy étant substituée le cas échéant par 1 à 5 restes de la série fluor et/ou chlore, alkoxy en C₁ à C₄, alkylthio en C₁ à C₄, acyloxy en C₁ à C₄, (alkoxy en C₁ à C₄)carbonyle, phényle, cyano ou nitro, ou des restes (alkyle en C₁ à C₆)-carbonyle qui sont substitués par 1 à 5 restes de la série (alkoxy en C₁ à C₄)carbonyle, cyano ou nitro, ou bien R² et R³ forment conjointement avec l'atome d'azote auquel ils sont liés un noyau de 4 à 6 chaînons,
R⁴ représente l'hydrogène, un reste phényle, alkyle en C₁ à C₆, qui est substitué le cas échéant par 1 à 5 restes de la série fluor et/ou chlore, alkoxy en C₁ à C₄, alkylthio en C₁ à C₄, acyloxy en C₁ à C₄, (alkoxy en C₁ à C₄)-carbonyle, phényle, cyano ou nitro,
Ar représente un reste phényle qui est substitué le cas échéant 1 à 4 fois identiques ou différentes par du fluor, du chlore ou du brome, un reste alkyle en C₁ à C₆, alcényle en C₂ à C₆, alcynyle en C₂ à C₆, les restes alkyle, alcényle ou alcynyle étant éventuellement substitués par 1 à 5 atomes de fluor et/ou de chlore, un reste alkoxy en C₁ à C₄ qui est substitué le cas échéant par 1 à 5 atomes de fluor et/ou de chlore, un reste thioalkyle en C₁ à C₄ qui est substitué le cas échéant par 1 à 5 atomes de fluor et/ou de chlore, un reste alkoxy en C₁ à C₄ qui est substitué le cas échéant par 1 à 5 atomes de fluor et/ou de chlore, un reste thioalkyle en C₁ à C₄ qui est substitué le cas échéant par 1 à 5 atomes de fluor et/ou de chlore, un reste acyloxy en C₁ à C₄, par un reste alkoxy en C₁ à C₆, alcénoxy en C₂ à C₆, alcynoxy en C₂ à C₆, les restes alkoxy, alcénoxy, alcynoxy étant éventuellement substitués par 1 à 5 atomes de fluor et/ou de chlore, par un reste thioalkyle en C₁ à C₆, thioalcényle en C₂ à C₆, thioalcynyle, les restes thioalkyle, thioalcényle, thioalcynyle étant éventuellement substitués par 1 à 5 atomes de fluor et/ou de chlore, par un reste (alkyle en C₁ à C₆)-carbonyloxy qui est substitué le cas échéant par 1 à 5 atomes de fluor et/ou de chlore, par un reste amino qui est substitué le cas échéant par 1 ou 2 restes alkyle identiques ou différents ayant 1 à 6 atomes de carbone, qui sont éventuellement substitués par 1 à 5 atomes de fluor et/ou de chlore, par un reste nitro ou cyano,
Y¹ représente le chlore ou le brome et
Y² représente le chlore ou le brome ou un groupe alkyle en C₁ à C₅ qui est substitué le cas échéant par 1 à 7 atomes identiques ou différents de fluor et/ou de chlore.

3. Procédé de production de 2-arylpyrroles substitués de formule générale (I), dans laquelle
R¹, R², R³, R⁴, Ar, Y¹ et Y² ont la définition indiquée dans la revendication 1, caractérisé en ce qu'on fait réagir des 2-arylpyrroles de formule (II) 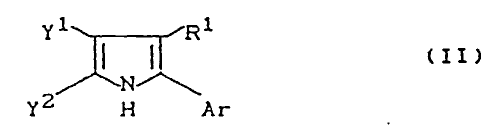 dans laquelle
R¹, Ar, Y¹ et Y² ont la définition indiquée ci-dessus, avec des composés de formule (III) 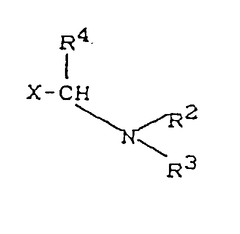 dans laquelle
R², R³ et R⁴ ont la définition indiquée ci-dessus, et
X représente un groupe partant anionique,
le cas échéant en présence de bases et/ou éventuellement en présence de diluants.

4. Compositions pesticides, caractérisées par une teneur en au moins un 2-arylpyrrole substitué de formule (I).

5. Procédé pour combattre des parasites animaux, caractérisé en ce qu'on fait agir des 2-arylpyrroles substitués de formule (I) sur des parasites animaux et/ou sur leur milieu.

6. Utilisation de 2-arylpyrroles substitués de formule (I) pour combattre des parasites animaux.

7. Procédé de préparation de compositions pesticides, caractérisé en ce qu'on mélange des 2-arylpyrroles substitués de formule (I) avec des diluants et/ou des agents tensio-actifs.
